Europäisches Patentamt

European Patent Office

Office européen des brevets

(19)

(11) Publication number: **0 161 107**
**A2**

## (12) EUROPEAN PATENT APPLICATION

(21) Application number: **85303174.8**

(22) Date of filing: **03.05.85**

(51) Int. Cl.⁴: **G 01 N 33/543**
**G 01 N 33/94, G 01 N 33/74**

(30) Priority: **08.05.84 GB 8411706**

(43) Date of publication of application:
**13.11.85 Bulletin 85/46**

(84) Designated Contracting States:
**AT BE CH DE FR GB IT LI LU NL SE**

(71) Applicant: **Farmos-Yhtymä Oy**
**P.O. Box 425**
**SF-20101 Turku 10(FI)**

(72) Inventor: **Hammond, Geoffrey Lewis**
**82 Ridout Street South Apartment 504**
**London Ontario(CA)**

(74) Representative: **Collier, Jeremy Austin Grey et al,**
**J.A.Kemp & Co. 14, South Square Gray's Inn**
**London WC1R 5EU(GB)**

(54) **Immunometric method for the determination of a hapten.**

(57) Haptens may be determined by a competitive binding immunometric technique in which a sample of hapten to be determined is incubated in solution with a labelled antibody to the hapten and a conjugate of the said hapten, the protein present in the said conjugate being different from that bound to the hapten during raising of the antibody. The conjugate is conveniently immobilized by binding of a non-hapten epitopes to an antibody which is linked to a solid support. The method may be calibrated by use of samples containing standard amounts of hapten.

Fig.1

## IMMUNOMETRIC METHOD FOR THE DETERMINATION OF A HAPTEN

This invention relates to the determination of haptens by a method in which labelled antibodies are used to measure the concentration of haptens in biological samples.

Immunoassay procedures are widely used to measure specific component molecules in biological samples, in particular blood samples from patients for prognostic, diagnostic, or other clinical purposes. At present, the most important immunochemical analytical methods employ the use of radioactive isotopes, molecules with fluorescent or bioluminescent properties, or enzymes which promote colour changes as the assay signal. These are generally referred to as the label.

The antibodies (immunoglobulins) used for immunochemical analyses have until recently been produced in the form of antisera in animals (e.g. rabbits or sheep) by immunization with an antigen, which is usually the analyte itself, or an analogue of the analyte, which promotes the production of antibodies that recognise the analyte.

In the case of small molecules, called haptens, the latter approach is particularly important, because molecules with a molecular weight of less than about 5,000 daltons are not inherently immunogenic and must be conjugated to a larger carrier molecule which is usually a protein, e.g. bovine serum albumin or thyroglobulin. The antibodies present in antisera raised against each conjugated haptens exhibit polyvalency; i.e. several populations of antibodies are present in the antisera, each of which recognises different aspects of the molecular configuration of the conjugated hapten. The antibodies therefore differ in their specificity. This diversity of antibodies present in a given antiserum may be advantageous in some respects, especially when the antigen is a large protein, as it facilitates the formation of large antibody-antigen complexes which are capable of precipitation. However, if pure preparations of specific antibodies are required, as in the case of immunometric assays, complex purification procedures must be employed and in general these are of limited efficiency. Recently, these problems have been overcome by the development of techniques for the large scale production of monoclonal hybridoma cells which secrete a single antibody with unique affinity and specificity for a given antigen. However, two or more antibodies which recognise different aspects of a hapten with a molecular weight of less than 2,000 daltons (e.g. steroids and many drugs) are unable simultaneously to bind to a single hapten molecule, and this has limited the use of labelled antibody (immunometric) immunoassays for most small molecular weight compounds.

In conventional immunoassays, a pure preparation of analyte, or an analogue of the analyte, is coupled to an appropriate label, the nature of which determines the method of detection used. For example, in a radioimmunoassay, a radioactive isotope is used as the label, and can be quantified in a liquid or crystal scintillation spectrometer.

This labelled analyte, or analyte analogue, may be incubated at a fixed concentration with a fixed amount of antibody that recognises the labelled analyte as well as the analyte itself. Competition between labelled and unlabelled analyte for the antibody binding site therefore occurs and the amount of labelled analyte bound by the antibody will be inversely proportional to the amount of unlabelled analyte present. The antibody-bound complexes formed are separated, for example, by immunoabsorption, physico-chemical adsorption or precipitation of either antibody-bound complexes or unbound analyte (labelled and unlabelled). Antibody-bound labelled analyte is then measured and a standard curve is constructed from known analyte concentrations (standards). The concentration of analyte in an unknown sample may be read off from this curve. In the case of immunoassays for small molecules, the labelled ligand is the labelled analyte or a derivative of the analyte (analogue) which may be covalently linked to a label. There is generally, however, a limit to the number of labels which may be linked to a small molecule

and this is largely determined by its size. Moreover, derivatization of small molecules or the introduction of labels of relatively large molecular size (e.g. $^{125}I$ or an enzyme) may alter the affinity of the antibody for the labelled analyte, relative to its affinity for unlabelled analyte, in such a way as to limit the usefulness of particular antibodies, e.g. antibodies which recognise the derivative side chain, and exhibit a so-called "bridge effect". The latter problem may be overcome by the use of antibodies (monoclonal or polyvalent) which display little affinity for the derivative side chain of the hapten, or by using a different derivative side chain to that used for constructing the antigen.

Immunometric assays involve the use of labelled antibodies in place of labelled analyte and are particularly useful for measurements of large molecules, e.g. proteins, which possess two or more discrete antigenic sites, or epitopes. In this type of assay, one or more antibody populations are used as "catching antibody", which may be immobilized on a solid-phase support, or may be immunoprecipitated with a second antibody specifically directed against it. The latter is only possible if the "catching antibody" is raised in a different species to the "labelled antibody". The "labelled antibody" is a pure preparation of one or more antibody populations, which preferably recognises a different epitope(s) to the "catching antibody", although this is not essential.

During incubation with standard amounts of analyte, or unknown concentrations of analyte in biological samples, a "sandwich" is formed between the "catching antibody", the analyte, and the "labelled antibody". Therefore in the presence of excess amounts of both "catching" and "labelled" antibodies, the amount of "labelled antibody" attached to such a "sandwich" is directly proportional to the amount of analyte present, and thereby provides the basis of a quantitative measurement.

Immunometric techniques offer numerous advantages over conventional competitive immunoassays, e.g. they obviate the need for pure preparations of labelled analyte. The use of excess reagents also reduces the necessity for precision pipetting of reagents, increases reaction kinetics and thereby reduces incubation times. However, the importance of this technique has only recently been fully appreciated, especially since the production of monoclonal antibodies has considerably simplified the problem of producing large quantities of antibodies with unique specificity. Nevertheless, the application of labelled antibody immunometric assays for small molecular weight haptens has been restricted, mainly because it may be sterically impossible for more than one antibody to interact with a small molecule at one time.

An attempt to overcome this problem has been made by immobilizing haptens conjugated to proteins (other than those used for antigenic purposes) to a "solid-phase"

support, see European Patent Publication No. 0028132.  This enables a competitive reaction for a labelled antibody binding site to occure between analyte and protein-conjugated-hapten; see European Patent Publication No. 0028133 and British Patent Specification No. 1550320.  Therefore under conditions where a fixed amount of protein-conjugated-hapten and labelled antibody are used, the amount of labelled antibody which is capable of binding to the protein-conjugated-hapten is inversely proportional to the amount of analyte present in standards or unknown samples. Although this type of protocol enables labelled antibodies to be used in immunoassays for small molecules, the amount of protein-conjugated-hapten added to the system in part influences the assay  range and sensitivity, and the relative amounts of protein-conjugated-hapten present in individual assay tubes, or cuvettes, determines (to a large extent) the assay precision.  This is a particular problem because the efficiency of passive, or active, adsorption of protein molecules to solid-phase supports is unreliable and difficult to control.  Moreover, large amounts of protein-conjugated hapten are required, the characteristics of which may alter between batches.

The above problems are overcome by the present invention, according to which a protein-conjugated hapten is prepared using a different protein (e.g. thyroglobulin) to that used to conjugate the hapten for the purpose of raising antibodies to the hapten (e.g. bovine serum albumin). The conjugated-hapten may be used for the affinity purification of sub-populations of antibodies from an antiserum which exhibits little recognition for areas of the hapten other than those present in the (different) hapten conjugate against which the antiserum was raised, if the same derivative side chain is used. In addition, a different side chain may also be used as this will eliminate binding of antibodies which recognise and bind to the side chain itself. However, the main function of the conjugated hapten is to act as a carrier of the hapten which is free to compete with analyte for labelled antibody binding sites in a liquid-phase, by virtue of the fact that the protein (thyroglobulin) may be separated immunochemically by an excess of antibodies directed against it (e.g. antithyroglobulin-antiserum), and which may be attached to a solid-phase support. In this way, a fixed amount of protein-conjugated-hapten is incubated together with a labelled antibody in the presence of variable amounts of standards or unknown serum samples. The amount of labelled antibody bound to the protein-conjugated hapten will be inversely proportional to the amount of analyte in the standards or unknown samples. The protein-conjugated-hapten-labelled antibody complexes are specifically removed from the incubates immunochemically by antibodies against the protein. These latter antibodies may be absorbed to the walls of the reaction vessel/tube or coupled to a solid phase support and separated physically after a short incubation period.

The present invention accordingly provides an immunometric method for the determination of a hapten which comprises

(I) forming a mixture containing (1) the hapten to be determined; (2) a labelled antibody to the said hapten raised against a conjugate of the said hapten with a macromolecular hapten carrier (i); and (3) a conjugate of the said hapten with a macromolecular hapten carrier (ii) different from said carrier (i), under conditions such that the hapten (1) and the hapten conjugate (3) become bound to the hapten binding sites of the labelled antibody (2);

(II) immobilizing the hapten conjugate (3) before, during or after the formation of the said mixture; and

(III) measuring the amount of label attached to the immobilized hapten conjugate (3) on the hapten to be determined (1).

The present invention also makes possible the affinity purification of polyvalent antibody populations from an antiserum which only recognises epitopes unique to the analyte and not the derivative side chain of the analyte hapten. This is achieved by linking the hapten to a different protein (e.g. thyroglobulin) to that used for immunization purposes (e.g. bovine serum albumin). The thyroglobulin-hapten is immobilized, on, for example, cyanogen bromide activated Sepharose 4B CL, and antisera raised against one hapten conjugate, e.g. bovine serum albumin-hapten, are incubated with another, immobilized hapten conjugate, e.g. thyroglobulin-hapten, so that antibodies to the hapten are themselves selectively immobilized. After extensive washing of the thyroglobulin-hapten affinity matrix, the antibodies which exhibit greatest affinity for the regions common to both hapten and analyte, and not the derivative side chain, are removed from the thyroglobulin-hapten affinity matrix by competition with excess analyte, which is subsequently removed from the antibody by dialysis or physical adsorption. This makes possible the production of antibodies having a high degree of specificity for the hapten in question.

In the present invention any type of protein or synthetic polypeptide capable of evoking an immune response can be used to conjugate the hapten. In addition, any small molecular compound, natural or synthetic (e.g. a drug, hormone or other biologically active low molecular weight compound), may be measured by the technique of the invention.

The separation method described (using, for example, immobilized antithyroglobulin antibody) may also be applied to other proteins or synthetic molecules and may be extended to include second antibody systems where an immobilized antibody may be used to bind and separate antibodies directed against the protein used to conjugate the hapten.

The invention includes within its scope an assay kit useful in the method of the invention comprising: (1) a labelled antibody to the said hapten; (2) a conjugate of the said hapten with a macromolecular hapten carrier different from that used in raising the labelled antibody (1); (3) means for immobilizing the said hapten conjugate (2); and (4) calibration standards of the hapten to be determined.

The means for immobilizing the hapten conjugate used in the assay method is preferably an antibody to the non-hapten epitopes of the said conjugate which is itself attached to a solid carrier or support, e.g. the inside of a plastics tube used for the assay or an inert powder such as kaolin. Alternatively, some other binding agent such as avidin can be used in which case avidin may be bound to a solid-phase and the hapten may be biotinylated, (i.e. bound to biotin), or _vice versa_ (the hapten may be linked to avidin and the solid-phase may be biotinylated).

The label used in the new method may be any label customarily used in immunometric and similar assay methods, e.g. a radioisotope, an enzyme, or a fluorescent, phosphorescent, luminescent, or spin-labelled molecule.

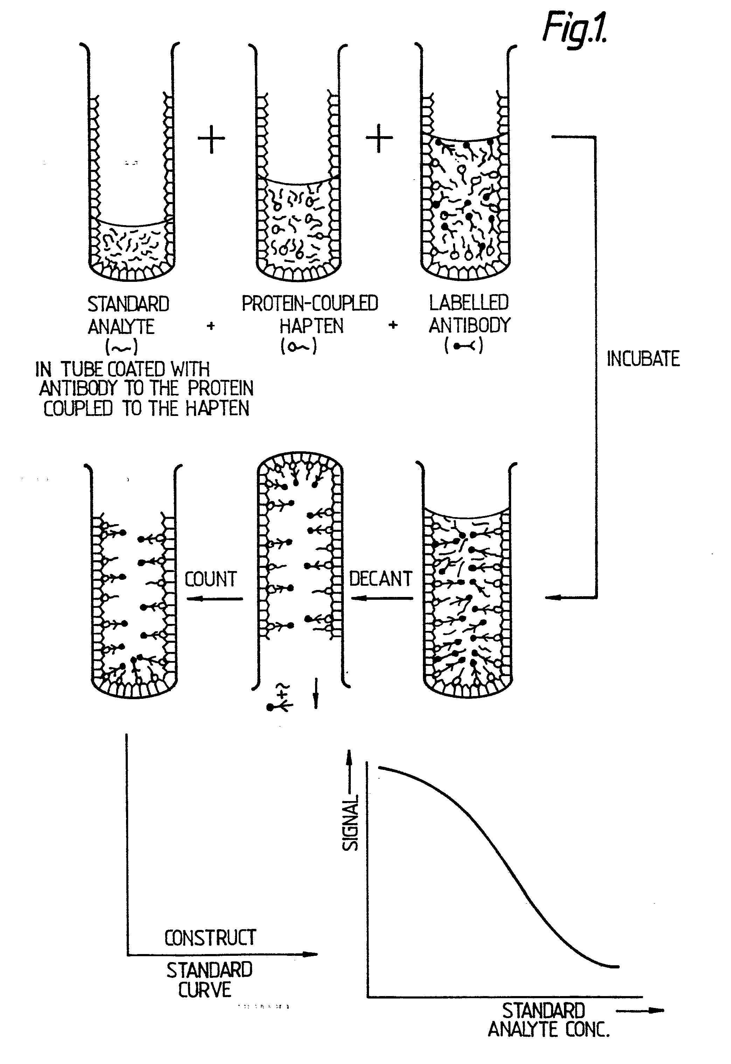

The method of the present invention may be operated in the manner shown diagrammatically in Figure 1 of the accompanying drawings. Initially a standard analyte, i.e. sample of the hapten to be determined having a known concentration, or an unknown analyte to be determined, is mixed with protein-coupled hapten and labelled antibody to the hapten (raised against a different protein from that in the protein-hapten conjugate used). Also provided is an antibody to the protein present in the protein-hapten conjugate immobilized by attachment to a solid support, shown as the interior surface of the tube (e.g. of polystyrene) used for the assay. During incubation the hapten conjugate attaches itself to the immobilized antibody. The hapten in the analyte and the hapten bound to the solid support compete for the labelled antibody so that part of the label is immobilized and the remainder (which attaches to the hapten in the analyte) is not. Decantation of the liquid phase accordingly leaves only the immobilized label which can then be measured. Using a series of analytes of known hapten concentration a standard analyte curve may be constructed from which the amount of hapten in an unknown sample can be read off.

The following Example illustrates the invention.

### Example

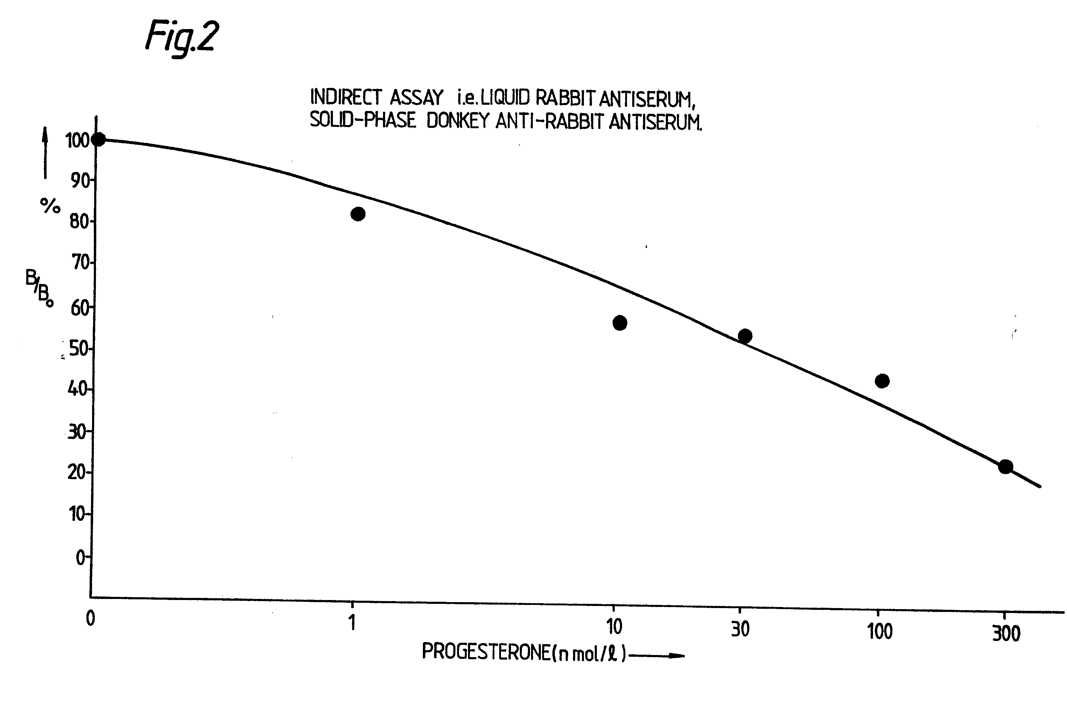

Duplicate 50 µl aliquots of human serum standards containing known amount of progesterone (0,1,10,30,100 and 300 nmol/1) were incubated (for 10 min at 20°C) in polystyrene tubes together with 100 µl of $^{125}$I-labelled monoclonal anti-progesterone antibody (100,000 cpm in assay buffer, i.e. phosphate-buffered saline containing phosphate (0.25 nmol/1), 0.9% NaCl, 0.625% lactose, 0.125% bovine gamma globulin, 0.05% sodium azide, pH 7.6). The monoclonal antibody had been produced against progesterone-11-hemisuccinate:bovine serum albumin and purified from culture medium by Protein A-Sepharose affinity chromatography. Aliquots (100 µl) of a progesterone-11-hemisuccinate:bovine thyroglobulin conjugate (1 nmol/l in assay buffer) were added to all but two tubes. These two tubes were used to monitor non-specific binding and contained 50 µl of the lowest progesterone standard serum (0 nmol/l), $^{125}$1-labelled monoclonal antibody and bovine thyroglobulin (100 µl, 1 nmol/l in assay buffer). After a further incubation (for 30 min at 20°C), 100 µl of rabbit antibovine thyroglobulin antiserum (1:500 in assay buffer) were added to all tubes, which were then vortex-mixed and incubated for 30 min at 20°C. Aliquots (1 ml) of kaolin-donkey anti-rabbit antiserum were added to all tubes and, after a further 30 min at 20°C, these were centrifuged (2,000g for 10 min). The supernatant was decanted and the residue was counted in a multi-well gamma counter. A standard curve was constructed, as shown in Figure 2. In this figure $B/B_0$ stands for the percentage binding of the standards (minus non-specific binding) compared with maximum binding (zero standard: $B_0$) minus non-specific binding.

If a similar assay is carried out using a sample containing an unknown amount of progesterone, determination of the percentage binding ($B/B_0$) obtained for the sample makes it possible to read off from this curve the concentration of progesterone in the sample.

- 13 -

CLAIMS

1. An immunometric method for the determination of a hapten which comprises:

(I) forming a mixture containing (I) the hapten to be determined; (2) a labelled antibody to the said hapten raised against a conjugate of the said hapten with a macromolecular hapten carrier (i); and (3) a conjugate of the said hapten with a macromolecular hapten carrier (ii) different from said carrier (i), under conditions such that the hapten (1) and the hapten conjugate (3) become bound to the hapten binding sites of the labelled antibody (2);

(II) immobilizing the hapten conjugate (3) before, during or after the formation of the said mixture; and

(III) measuring the amount of label attached to the immobilized hapten conjugate (3) on the hapten to be determined (1).

2. A method according to claim 1 wherein, before the label is measured, the labelled immobilized hapten conjugate (3) is separated from the labelled hapten (1).

3. A method according to claim 1 or 2 wherein the hapten conjugate (3) is immobilized by binding to a binding agent with high affinity therefor.

4. A method according to claim 3 wherein the said binding agent is an antibody against the macromolecular hapten carrier (ii) or an avidin-biotin system.

5. A method according to claim 3 or 4 wherein the said binding agent is itself immobilized by binding to a solid carrier.

6. A method according to claim 5 wherein the said solid carrier is kaolin.

7. A method according to any of claims 1 to 6 wherein the macromolecular hapten carriers (i) and (ii) are both different proteins or synthetic polypeptides.

8. A method according to any of claims 1 to 7 wherein the said label is a radioisotope, an enzyme, or a fluorescent, phosphorescent, luminescent, or spin-labelled molecule.

9. A method according to any of claims 1 to 8 wherein the said hapten is a natural or synthetic drug, hormone or other biologically active low molecular weight compound.

10. An assay kit useful for determining a hapten by the method of any of the preceding claims comprising: (1) a labelled antibody to the said hapten; (2) a conjugate of the said hapten with a macromolecular hapten carrier different from that used in raising the labelled antibody (1); (3) means for immobilizing the said hapten conjugate (2); and (4) calibration standards of the hapten to be determined.

11. An assay kit according to claim 10 wherein the means (3) for immobilizing the hapten conjugate (2) comprises an antibody to the said macromolecular hapten carrier bound directly or indirectly to a solid carrier.

1/2

0161107

Fig.1.

STANDARD ANALYTE (∿) + PROTEIN-COUPLED HAPTEN (o∿) + LABELLED ANTIBODY (•‹)

IN TUBE COATED WITH ANTIBODY TO THE PROTEIN COUPLED TO THE HAPTEN

INCUBATE

COUNT ← DECANT ←

CONSTRUCT STANDARD CURVE

SIGNAL →

STANDARD ANALYTE CONC. →

Fig.2

INDIRECT ASSAY i.e. LIQUID RABBIT ANTISERUM,
SOLID-PHASE DONKEY ANTI-RABBIT ANTISERUM.